# EUROPEAN PATENT APPLICATION

(11) **EP 4 443 167 A1**
(43) Date of publication of application: **09.10.2024**
(21) Application number: 22901224.0
(22) Date of filing: 28.11.2022
(51) Int. Cl.: G01N 35/10

(54) **BLOOD ANALYZER, BLOOD ANALYSIS METHOD AND PROGRAM**

(30) Priority: 03.12.2021 JP 2021196633
(71) Applicant: HORIBA, Ltd., Minami-ku Kyoto-shi Kyoto 601-8510 (JP)
(72) Inventor: ISSHIKI Ryota, Kyoto-shi, Kyoto 601-8510 (JP); IKEDA Motohide, Kyoto-shi, Kyoto 601-8510 (JP); OSAWA Kenta, Kyoto-shi, Kyoto 601-8510 (JP)
(74) Representative: Isarpatent
(86) International application number: PCT/JP2022/043724
(87) International publication number: WO 2023/100791

(57) **Abstract**

A blood analysis apparatus includes: a container information acquisition portion that acquires container information on the type of sample container to be used; a needle that sucks and discharges a blood sample contained in the sample container; a blood analysis portion having a chamber that receives the blood sample sucked from the sample container and discharged into it with the needle; and a control portion that controls suction and discharge of the blood sample with the needle based on the container information. The control portion sets the amount of blood sample to be sucked with the needle based on the container information.

## Description

### Technical Field

The present invention relates to a blood analysis apparatus for analyzing a blood sample stored in a sample container, to a blood analysis method, and to a program.

### Background Art

Conventionally, a blood collection apparatus has been proposed that collects blood by depressurizing a vacuum blood collection chamber in a housing and applying a predetermined negative pressure to a blood bag (see, for example, Patent Document 1 identified below). In recent years, apparatuses for blood analysis have also been proposed in which a blood collection tube is, with a lid closed, set in the apparatus body (see, for example, Non-patent Document 1).

### Citation List

### Patent Literature

Patent Document 1: Japanese Examined Utility Model Application Publication No. H6-28112.

### Non-Patent Literature

Non-Patent Document 1: Kohden Partners Vol.6, published by Nihon Kohden Corp.

### Summary of Invention

### Technical Problem

A blood collection tube that can be set in an apparatus body with the lid closed is here referred to as a sample container of a closed type. When a sample container of a closed type is used, piercing the lid of the sample container with a needle permits a blood sample contained in the sample container to be sucked with the needle. When suction of the blood sample is complete, the needle is taken out of the sample container. Then, the blood sample is discharged from the needle into a container (chamber) in a blood analysis portion such as a blood cell counting portion. This permits blood analysis (for example, blood cell counting) to be performed in the blood analysis portion.

However, when using a sample container of a closed type, removing the needle from the sample container after suction of the blood sample may result in the blood sample in the needle being pushed inward of it due to a pressure difference (pressure variation) between inside and outside the sample container. For example, if the pressure inside the sample container is higher than the external pressure, the blood sample inside the needle is pushed inward of the needle.

In this case, even when the blood sample is intended to be pushed out from the needle by a defined amount, the air trapped in a tip of the needle is pushed out before the blood sample; thus, the amount of the discharged blood sample is smaller than the defined amount by the amount of air that has been pushed out. As a result, the blood analysis portion cannot accurately analyze the blood sample discharged into the chamber.

On the other hand, the blood collection tube that is set in the apparatus body with the lid removed is here referred to as a sample container of an open type. When a sample container of an open type is used, there is no lid that closes the sample container, and thus no pressure difference arises between inside and outside the sample container. Thus, after suction of the blood sample, removing the needle from the sample container causes no movement of the blood sample in the needle due to a pressure difference as mentioned above. Thus, when using a sample container of an open type, it is not appropriate to employ a method similar to one for a sample container of a closed type for accurate blood sample analysis.

The present invention is devised to solve the above-mentioned problems and aims at providing a blood analysis apparatus, a blood analysis method, and a program that permit accurate analysis of a blood sample regardless of the type of sample container used (closed type, open type).

### Solution to Problem

According to one aspect of the present invention, a blood analysis apparatus includes: a container information acquisition portion that acquires container information on the type of a sample container to be used; a needle that sucks and discharges a blood sample contained in the sample container; a blood analysis portion having a chamber that receives the blood sample sucked from the sample container and discharged into it with the needle; and a control portion that controls suction and discharge of the blood sample with the needle based on the container information. The control portion sets the amount of blood sample to be sucked with the needle based on the container information.

According to another aspect of the present invention, a blood analysis method includes: a suction amount setting step of setting an amount of blood sample to be sucked with a needle based on container information on the type of sample container to be used; a suction step of sucking with the needle the set suction amount of the blood sample from the sample container; a discharging step of discharging at least part of the sucked blood sample from the needle into a chamber in a blood analysis portion; and an analyzing step of analyzing the blood sample discharged in the chamber.

According to yet another aspect of the present invention, a program makes a computer execute the blood analysis method described above.

According to still another aspect of the present invention, a program makes a computer execute: a suction amount setting step of setting an amount of blood sample to be sucked with a needle based on container information on the type of a sample container to be used, and an output step of outputting information on the set suction amount to a blood analysis apparatus.

### Advantageous Effects of Invention

According to the present invention, it is possible to analyze a blood sample accurately regardless of the type of sample container used (closed type, open type).

### Brief Description of Drawings

[Fig. 1] Fig. 1 is a perspective view showing an exterior construction of a blood analysis apparatus according to one embodiment of the present invention.
[Fig. 2] Fig. 2 is an illustrative diagram schematically showing the internal configuration of the blood analysis apparatus.
[Fig. 3] Fig. 3 is a side view of a sample container of a closed type (first type).
[Fig. 4] Fig. 4 is a perspective view of a sample container of an open type (second type).
[Fig. 5] Fig. 5 is a perspective view of an adapter in which the sample container of the first type is inserted.
[Fig. 6] Fig. 6 is a perspective view of an adapter in which the sample container of the second type is inserted.
[Fig. 7] Fig. 7 is a perspective view showing one step involved in loading the sample container of the first type in an apparatus body.
[Fig. 8] Fig. 8 is a plan view of the interior of the sample container loading portion with the sample container of the first type loaded in the apparatus body.
[Fig. 9] Fig. 9 is a perspective view of the adapter in Fig. 6 inserted in the adapter in Fig. 5.
[Fig. 10] Fig. 10 is a plan view of the interior of the sample container loading portion with the sample container of the second type loaded in the apparatus body using the adapter in Fig. 9.
[Fig. 11] Fig. 11 is a flow chart showing a sequence of operation on the blood analysis apparatus.
[Fig. 12] Fig. 12 is a diagram schematically illustrating the variation, as observed in the needle, of the blood sample collected from the sample container of the first type.
[Fig. 13] Fig. 13 is a diagram schematically illustrating the variation, as observed in the needle, of the blood sample collected from the sample container of the second type.
[Fig. 14] Fig. 14 is a perspective view showing another construction of the blood analysis apparatus.
[Fig. 15] Fig. 15 is a block diagram showing an outline of the configuration of a blood analysis system.

### Description of Embodiments

An illustrative embodiment of the present invention will be described below with reference to the accompanying drawings.

### [ 1. Outline of a Blood Analysis Apparatus ]

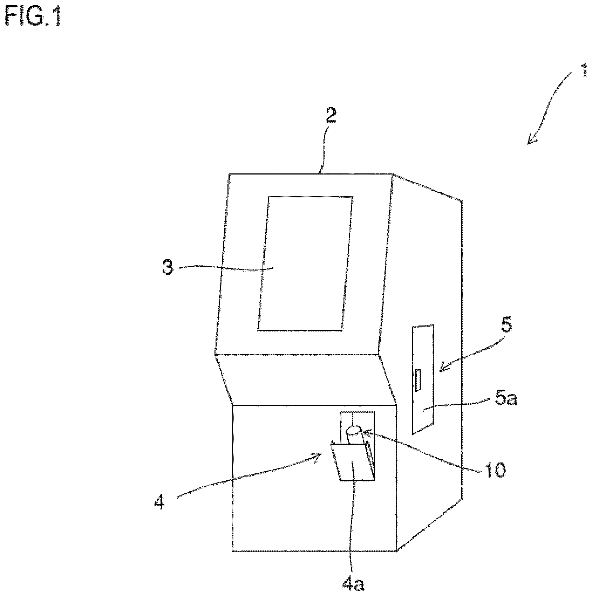

Fig. 1 is a perspective view showing an exterior construction of a blood analysis apparatus 1 according to the embodiment. The blood analysis apparatus 1 includes a display portion 3 in an upper part of the front face of an apparatus body 2. The display portion 3 is configured with, for example, a liquid crystal display device with a touch panel, and displays the results of blood analysis and the like and also accepts the input of various kinds of information by an operator (e.g., a medical professional).

In a lower part of the apparatus body 2, a sample container loading portion 4 is provided. Opening a cover 4a of the sample container loading portion 4, then setting a sample container 10 containing a blood sample, and then closing the cover 4a permits the sample container 10 to be loaded in the apparatus body 2. The sample container 10 is loaded in the apparatus body 2 with an adapter that suits the type of sample container 10 attached to it. The type of sample container 10 and the detail of the adapter to be used will be described later.

In a side part of the apparatus body 2, a reagent container loading portion 5 is provided. Opening a door 5a of the reagent container loading portion 5 permits the refilling with a reagent (e.g., a reagent for immunoassay) used in blood analysis.

### [ 2. Internal Configuration of the Blood Analysis Apparatus ]

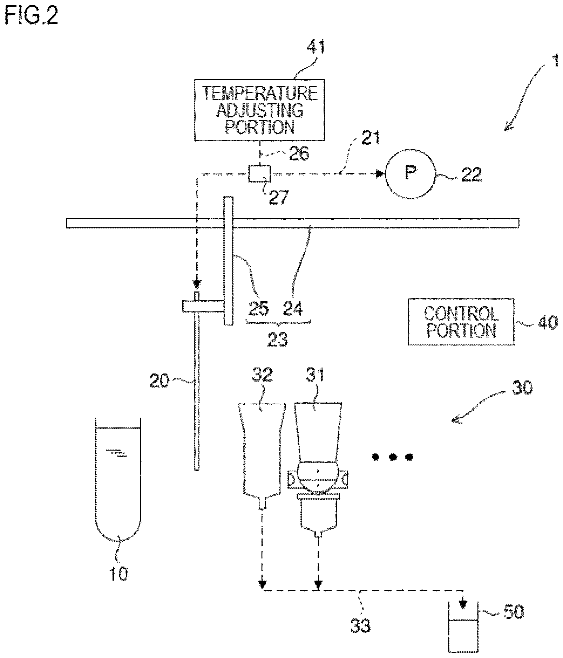

Fig. 2 is an illustrative diagram schematically showing the internal configuration of the blood analysis apparatus 1. The blood analysis apparatus 1 includes a needle 20, a blood analysis portion 30, and a control portion 40. The control portion 40 is configured with, for example, a central arithmetic processor (computer) generally called a CPU (central processing unit) and controls the operation of different parts in the blood analysis apparatus 1.

The needle 20 sucks and discharges the blood sample contained in the sample container 10. The needle 20, as necessary, pierces the lid of the sample container 10 to suck and discharge the blood sample. The base end of the needle 20 is connected to a quantitative syringe 22 via a first pipe 21 as indicated by broken lines and an electromagnetic valve device (not shown). As the control portion 40 drives the quantitative syringe 22 and the electromagnetic valve device, the needle 20 sucks and discharges the blood sample.

The needle 20 is moved by a probe unit 23 in horizontal and vertical directions. The probe unit 23 is configured to include a horizontal moving mechanism 24 and a vertical moving mechanism 25. The horizontal moving mechanism 24 and the vertical moving mechanism 25 include, for example, an endless belt, driving and driven rollers that run the endless belt, and the like. As the control portion 40 drives the driving rollers in the horizontal and vertical moving mechanisms 24 and 25, the endless belt in the horizontal moving mechanism 24 runs in the horizontal direction, and the endless belt in the vertical moving mechanism 25 in the vertical direction. This permits the needle 20 supported on the vertical moving mechanism 25 to move in the horizontal and vertical directions.

The horizontal moving mechanism 24 and the vertical moving mechanism 25 may be configured with a feed screw mechanism. The feed screw mechanism rotates a feed screw with a stepping motor and thereby moves an engaging portion that engages with the feed screw in the horizontal or vertical direction. Thus, coupling the vertical moving mechanism 25 to the engaging portion of the horizontal moving mechanism 24 and making the engaging portion of the vertical moving mechanism 25 hold the needle 20 permits the needle 20 to be moved in the horizontal and vertical directions.

The blood analysis portion 30 analyzes blood as by counting blood cells and performing immunoassay. The blood analysis portion 30 includes a chamber 31 (container) that receives a blood sample sucked from the sample container 10 and discharged into it with the needle 20. Inside a chamber 31 for blood cell counting, a counting device is provided. The counting device can carry out, according to the type of blood cells to be counted, various measurement methods including those based on impedance, flow cytometry, and focused flow impedance. The control portion 40 can process the measurement data acquired in the counting device to create a frequency distribution or the like. The counting device may be provided outside the chamber 31 and connected to the chamber 31.

The chamber 31 is provided to suit the type of blood cells to be counted. For example, as the chamber 31, an RBC chamber and a WBC chamber are separately provided. The RBC chamber is provided for counting red blood cells. The WBC chamber is provided for counting white blood cells and analyzing HGB (hemoglobin).

As the chamber 31, a BASO chamber and an LMNE chamber may be further provided. The BASO chamber is a chamber for counting basophils contained in white blood cells. The LMNE chamber is a chamber for counting lymphocytes, monocytes, neutrophils, and eosinophils contained in white blood cells. The LMNE chamber has "LMNE" derived from the initial letters of lymphocytes, monocytes, neutrophils, and eosinophils.

As the chamber 31, a CRP measuring chamber may be further provided. The CRP measuring chamber is a chamber configured to be able to optically measure the CRP value by latex agglutination. CRP is an abbreviation of C-reactive protein. The CRP measurement chamber includes a light emitter and a light detector for CRP measurement on a lower wall surface of the chamber and is designed to be able to stir the liquid contained in it as necessary.

Near the CRP measuring chamber, a plurality of reagent containers (not shown) are provided. The reagent containers contain a hemolytic reagent, a buffer solution, an antihuman CRP sensitized latex immunoassay reagent, and the like. In performing CRP measurement, reagents are sucked from the reagent containers with the needle 20 with appropriate timing and are discharged into the CRP measurement chamber.

The blood analysis portion 30 includes a cleaning container 32. The cleaning container 32 is provided to remove blood adhering to the needle 20 and to discard an excess of the blood sample in the needle 20.

To lower end parts of the chamber 31 and the cleaning container 32 described above, a discharge pipe 33 is connected as indicated by broken lines. Waste liquid in the chamber 31 and the cleaning container 32 is, as a discharge portion (not shown) is driven, sent to a waste liquid container 50 via an electromagnetic valve device (not shown). The discharge portion is configured with a discharge syringe or a discharge pump.

When the sample container 10 containing a blood sample is loaded in the sample container loading portion 4 (see Fig. 1) in the apparatus body 2, the control portion 40 drives the probe unit 23 to move the needle 20 in the horizontal and vertical directions. This permits the needle 20 to move into and out of each of the sample container 10, the chamber 31, and the reagent container. In addition, the control portion 40 can drive the quantitative syringe 22 to suck the blood sample or the reagent through the needle 20 or to discharge the blood sample or the reagent from the needle 20. Through such operations, blood analysis (blood cell counting and immunoassay) is performed in the blood analysis portion 30.

The blood analysis apparatus 1 further includes a temperature adjusting portion 41. The temperature adjusting portion 41 is connected, for example, to a second pipe 26 that branches from the first pipe 21. In a branch portion between the first pipe 21 and the second pipe 26, a switching valve 27 is provided to switch the flow path. The temperature adjusting portion 41 warms a diluent contained in a tank (also referred to as a buffer tank) with a heater wound around it. The warmed diluent flows into the first pipe 21 via the second pipe 26 and the switching valve 27, and is then discharged into the chamber 31 through the needle 20. Here, also the blood sample previously sucked from the sample container 10 with the needle 20 is discharged into the chamber 31 together with the warmed diluent. Thus, in the chamber 31, the blood sample is warmed to a desired temperature by mixing with the warmed diluent. The temperature adjusting portion 41 may be configured to warm the blood sample in the chamber 31 by directly heating the chamber 31 with a heater or the like. By warming the blood sample to a desired temperature in this way, it is possible to prevent the coagulation of a blood sample that coagulates under cool or cold conditions.

### [ 3. Type of Sample Container ]

Next, a description will be given of the sample container 10 to be used in the blood analysis apparatus 1. In this embodiment, as the sample container 10, one of a closed type (first type) and one of an open type (second type) can both be used. In the following description, wherever necessary to distinguish between sample containers of a closed type and an open type, the one of a closed type is referred to as the sample container 10P, and the one of an open type is referred to as the sample container 10Q.

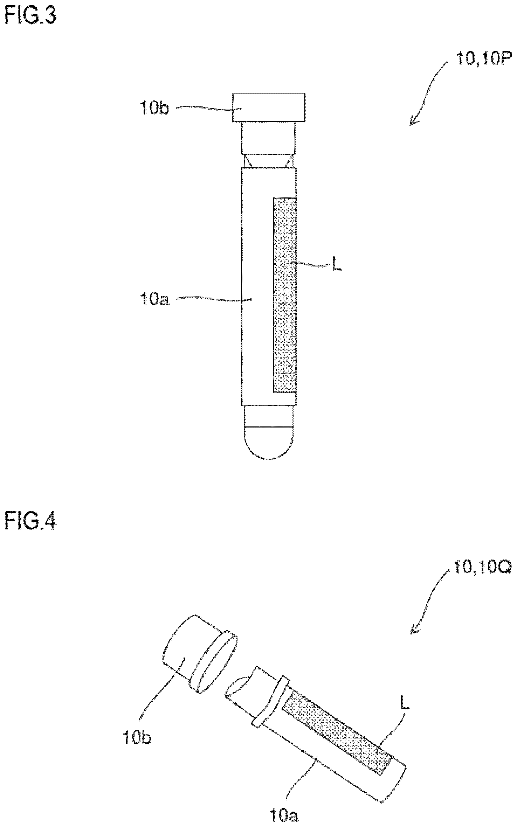

Fig. 3 is a side view of the sample container 10P of a closed type. The sample container 10P is a container that permits blood to be collected with a lid 10b fitted to a container body 10a. The sample container 10P has a negative pressure inside it and is referred to also as a vacuum blood collection tube. Sticking a syringe needle attached to a container holder (not shown) into a vein of a patient, then inserting the sample container 10 into the container holder, and then piercing the lid 10b with the needle inside the container holder permits the patient's blood to be sucked into and collected in the sample container 10P under the negative pressure mentioned above. The sample container 10P can accommodate, for example, 1 to 5 mL of blood.

To perform blood analysis using the sample container 10P, with the lid 10b fitted to the container body 10a, the sample container 10P is loaded in the apparatus body 2 (sample container loading portion 4). Then, with the needle 20 (see Fig. 2) pierced into the lid 10b, the blood inside is sucked. That is, the needle 20 pierces the lid 10b of the sample container 10P to suck the blood sample contained in the sample container 10P. In this way, an operator makes no contact with the patient's blood during blood analysis; thus, the sample container 10P of a closed type is effective in preventing infection.

Fig. 4 is a perspective view of the sample container 10Q of an open type. The sample container 10Q is a container with which the blood collected from a patient is directly sent into its container body 10a with the lid 10b removed. After the blood is collected, the lid 10b is fitted to the container body 10a. The sample container 10Q can accommodate, for example, 0.2 to 0.5 mL of blood. For example, when blood has to be collected from an elderly person, an infant, or a patient with whom ordinary blood collection is difficult, only a small amount of blood can be collected. In that case, the sample container 10Q is used for blood collection.

To perform blood analysis using the sample container 10Q, with the lid 10b removed from it, the sample container 10Q (thus only the container body 10a) is loaded in the apparatus body 2 (sample container loading portion 4). The needle 20 is inserted into the container body 10a and sucks the blood inside. Thus, the sample container 10Q of an open type can be understood as a container with which, with the lid 10b of the sample container 10Q removed, the needle 20 is inserted into the sample container 10Q to suck the blood sample directly.

As shown in Figs. 3 and 4, a label L may be attached to the surface of the container body 10a. The label L is printed with peripheral information on the sample container 10. The peripheral information includes registration information such as a registration number of the patient who presented blood, information on a reagent in the sample container 10 (e.g., whether an anticoagulant is contained), and identification information unique to each sample container 10 for distinguishing individual sample containers 10. The identification information includes manufacturing information such as the manufacturing number and the manufacturer of the sample container 10 and information on the type of sample container 10 (whether it is of a closed type or of an open type). While at least part of the peripheral information is represented by, for example, a bar code (one-dimensional code), it may be represented by, instead of a bar code, a two-dimensional code such as a QR code (a registered trademark).

### [ 4. Detection of the Adapter and the Sample Container ]

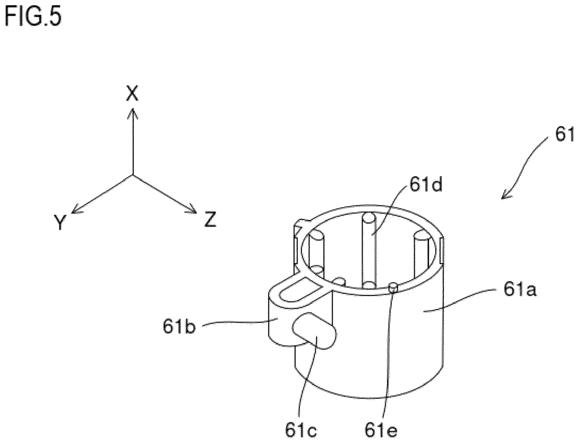

The sample container 10P of a closed type is inserted in an adapter 61 as shown in Fig. 5 when it is loaded in the apparatus body 2. The adapter 61 is configured to have a cylindrical main body 61a, a support portion 61b, a projection 61c, and a plurality of ribs 61d. The sample container 10P is inserted in the main body 61a.

The support portion 61b is provided so as to project radially from the outer circumferential face of the main body 61a. Assuming that the axial direction of the cylindrical main body 61a is the X-direction, the support portion 61b is formed in a U-shape as seen from the X-direction, though there is no particular limitation on the shape of the support portion 61b. Assuming that the projection direction of the support portion 61b from the outer circumferential face of the main body 61a is the Y-direction, the projection 61c is provided so as to project from the support portion 61b in the Z-direction perpendicular to the XY plane.

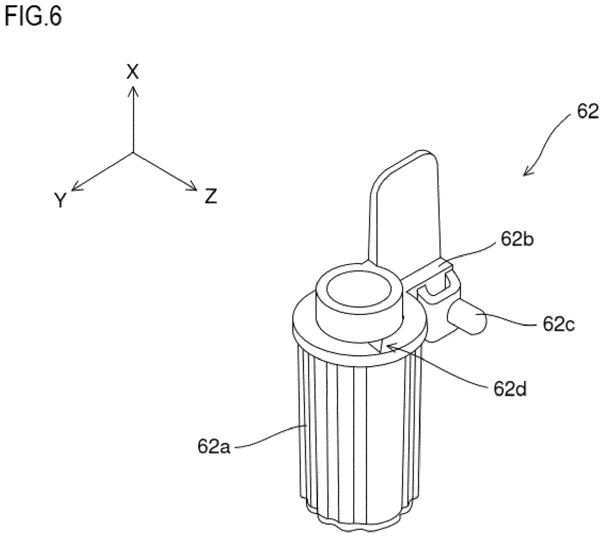

The plurality of ribs 61d are provided on the inner circumferential face of the main body 61a so as to extend along the X direction, at intervals from each other in the circumferential direction. When inserted into the main body 61a, the sample container 10P makes contact with the plurality of ribs 61d. This permits the sample container 10P to be held in the main body 61a. On the upper face of the main body 61a at its end in the X directional, a boss 61e is provided for alignment relative to an adapter 62 (see Fig. 6), which will be described later.

By contrast, the sample container 10Q of an open type is loaded in the apparatus body 2 using both the adapter 62 shown in Fig. 6 and the adapter 61 shown in Fig. 5. The adapter 62 is configured to have a main body 62a having a cylindrical inner circumferential face, a support portion 62b, and a projection 62c. The sample container 10Q is inserted and held in the main body 62a.

In this embodiment, the main body 62a has two-step diameters, though no limitation to that shape is meant. When the axial direction of the cylindrical main body 62a is the X-direction the same as in the coordinate system of the adapter 61, a mark 62d for alignment relative to the adapter 61 (see Fig. 5) is provided in an upper part of the main body 62a in the X-direction.

The support portion 62b is provided so as to project radially from the outer circumferential face of the main body 62a. The projection direction of the support portion 62b relative to the main body 62a is a direction perpendicular to the X direction, but is a direction opposite to the direction (-Y direction) in which the support portion 61b of the adapter 61 projects. The support portion 62b is formed in a shape of a plurality of members such as flat plates combined together, though there is no particular limitation on the shape of the support portion 62b. The projection 62c projects from the support portion 62b, and its projection direction is the same (Z direction) as the projection direction of the projection 61c of the adapter 61.

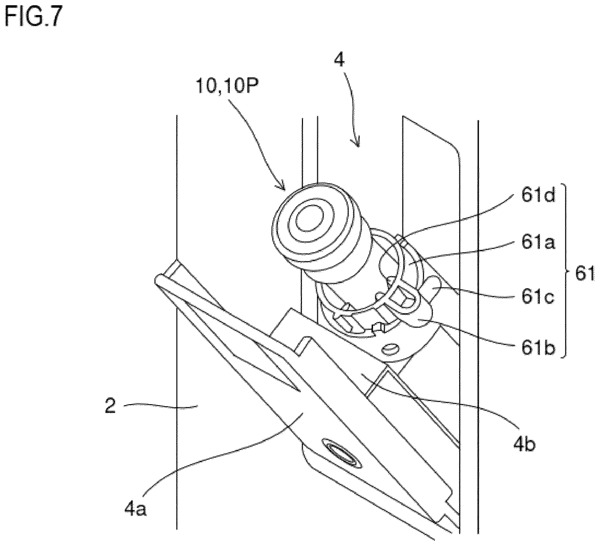

When the sample container 10P of a closed type is loaded in the apparatus body 2, as shown in Fig. 7, the sample container 10P inserted in the adapter 61 is placed on the support stand 4b inside the cover 4a, and then the cover 4a is closed.

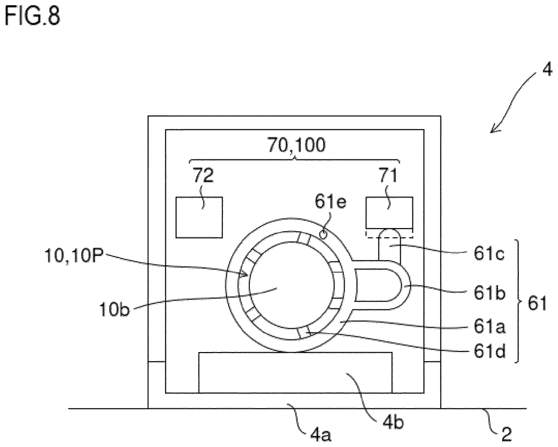

Fig. 8 is a plan view of the interior of the sample container loading portion 4 with the cover 4a closed and the sample container 10P loaded. The sample container loading portion 4 includes an adapter sensing portion 70. The adapter sensing portion 70 includes a first switch 71 and a second switch 72, which are arranged horizontally away from each other.

When the cover 4a is closed with the sample container 10P placed on the support stand 4b, the projection 61c on the adapter 61 presses the first switch 71. On the other hand, the second switch 72 remains unpressed. Thus, the adapter sensing portion 70 can sense that the adapter 61 is used and that the sample container 10P of a closed type is loaded.

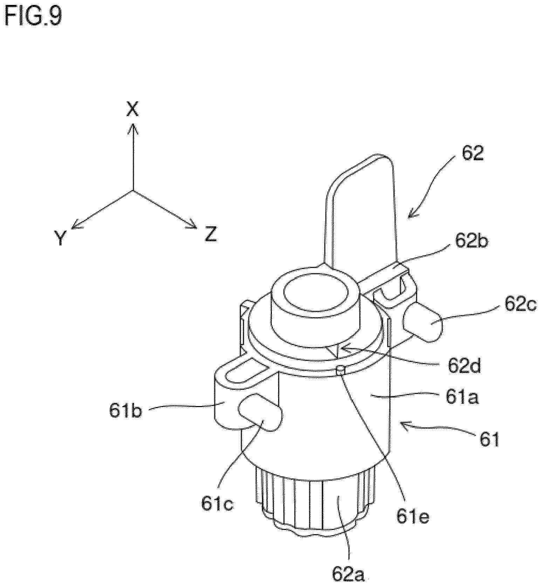

On the other hand, when the sample container 10Q of an open type is loaded in the apparatus body 2, as shown in Fig. 9, the main body 62a of the adapter 62 is first inserted into the main body 61a of the adapter 61. Here, the boss 61e on the adapter 61 and the mark 62d on the adapter 62 are aligned in position. In this state, the projection 61c and projection 62c are positioned apart from each other in the Y direction but parallel to each other in the Z direction. Then, the sample container 10Q is inserted into the main body 62a of the adapter 62. It is also possible to insert the sample container 10Q into the adapter 62 first, and then insert the adapter 62 into the adapter 61. Then, the sample container 10Q and adapters 61 and 62 are, as a set, placed on the support stand 4b inside the cover 4a, and the cover 4a is closed.

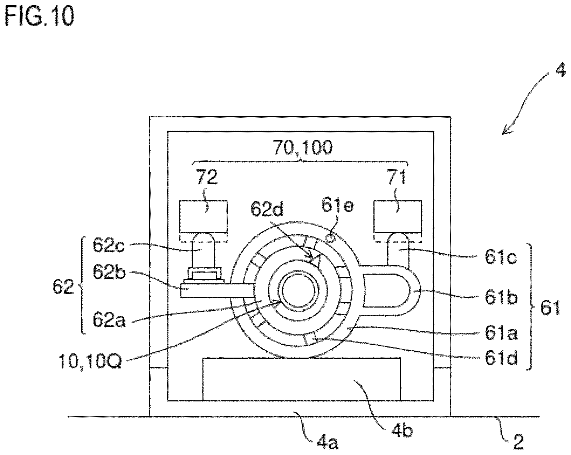

Fig. 10 is a plan view of the inside of the sample container loading portion 4 with the cover 4a closed and the sample container 10Q loaded. When the cover 4a is closed with the sample container 10Q placed on the support stand 4b, the projection 61c on the adapter 61 presses the first switch 71, and the projection 62c on the adapter 62 presses the second switch 72. Thus, the adapter sensing portion 70 can sense that the adapters 61 and 62 are in use and that the sample container 10Q of an open type is loaded.

In this way, the adapter sensing portion 70 senses whether the sample container 10 in use is of a closed type or of an open type, and acquires information on it (information on the type of sample container 10). Thus, it can be said that the adapter sensing portion 70 constitutes a container information acquisition portion 100 that acquires information on the type of sample container 10 in use. The blood analysis apparatus 1 includes such a container information acquisition portion 100.

When the sample container in use is the sample container 10P of a closed type, the adapter 61 is used alone, and when the sample container in use is the sample container 10Q of an open type, the adapters 61 and 62 are used together. Thus, it can also be said that, when the sample container 10 is attached to at least one adapter determined according to the type of sample container 10 and loaded in the apparatus body 2, the adapter sensing portion 70 senses the adapter and thereby acquires information on the type of sample container 10. In this way, by using the adapter sensing portion 70 as the container information acquisition portion 100, it is possible, based on sensing of the adapter used, to reliably acquire information on the type of sample container 10 in use.

### [ 5. Control of Suction and Discharge of a Blood Sample with the Needle ]

In this embodiment, the control portion 40 controls the suction and discharge of a blood sample with the needle 20 based on container information acquired in the container information acquisition portion 100 (adapter sensing portion 70). Operation in this control will be described below.

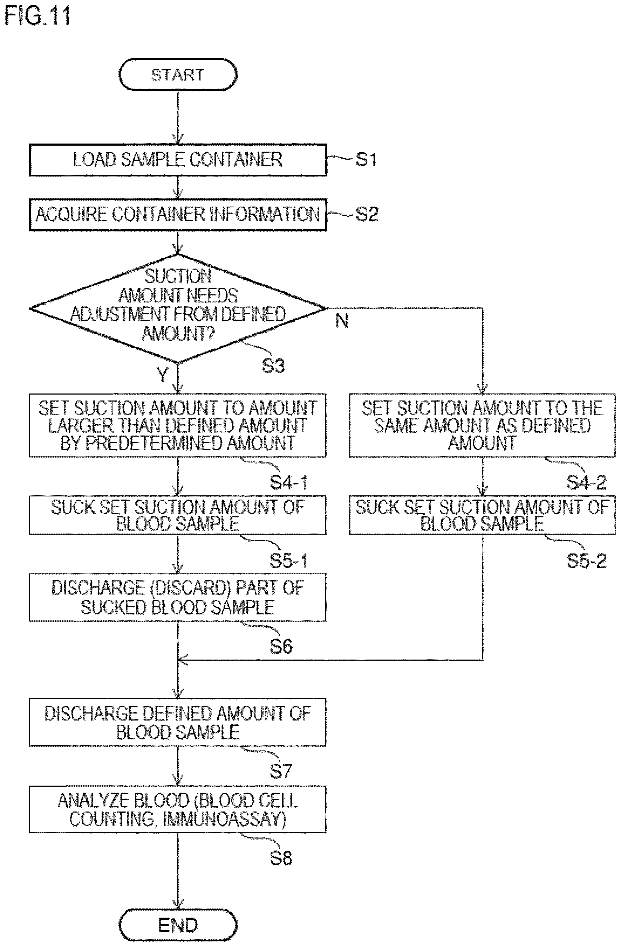

Fig. 11 is a flow chart showing the procedure of operation in the blood analysis apparatus 1. First, when a sample container 10 (of a closed type or of an open type) inserted in a corresponding adapter is loaded in the sample container loading portion 4 in the apparatus body 2 (S1), the adapter sensing portion 70 senses the adapter to check whether the loaded sample container 10 is of a closed type or of an open type, and acquires container information on the type of sample container 10 loaded (S2; an information acquisition step).

Next, the control portion 40, based on the container information (on the type of sample container 10) acquired in S2, judges whether the amount of blood sample to be sucked with the needle 20 needs to be adjusted from a prescribed defined amount (for example, 10 µL) (S3). Then, the control portion 40 sets the suction amount based on the judgment result in S3 (S4-1, S4-2; a suction amount setting step).

For example, if it is recognized from the above container information that the sample container 10 is a sample container 10P of a closed type, the control portion 40 judges that the suction amount V of the blood sample with the needle 20 needs to be adjusted from the defined amount Vs (S3). Then, the control portion 40 sets the suction amount V to an amount larger than the defined amount Vs by a predetermined amount Vm (S4-1). The defined amount Vs and the predetermined amount Vm can be set freely. For example, the defined amount Vs can be appropriately set to an amount needed for blood analysis (such as counting of red blood cells or white blood cells (5 types), measurement of the CRP values, or the like). Also the predetermined amount Vm can be set appropriately based on the experience of use of the blood analysis apparatus 1 by an operator (e.g., a medical professional) or based on predictions based on the use experience.

By contrast, if, based on the above container information, it is recognized that the sample container 10 is a sample container 10Q of an open type, the control portion 40 judges that there is no need to adjust, from the defined amount Vs, the suction amount V of the blood sample with the needle 20 (S3). Then, the control portion 40 sets the suction amount V to the same amount as the defined amount Vs (S4-2).

Next, the control portion 40 drives the quantitative syringe 22 (see Fig. 2) to suck as much of the blood sample from the sample container 10 through the needle 20 as the suction amount V set in S4-1 or S4-2 (S5-1, S5-2; a suction step).

That is, if the sample container 10 is of a closed type, the needle 20 sucks the blood sample from the sample container 10P by the suction amount V = Vs + Vm set in S4-1 (S5-1). By contrast, if the sample container 10 is of an open type, the needle 20 sucks the blood sample from the sample container 10Q by the suction amount V = Vs that is set in S4-2 (S5-2). Here, the suction amount V can be easily controlled, for example, by controlling the duration for which the control portion 40 drives the quantitative syringe 22. In steps S5-1 and S5-2, when the sucking of the blood sample is complete, the control portion 40 drives the probe unit 23 (see Fig. 2) to take the needle 20 out of and move it away from the sample container 10.

Next, the control portion 40 drives the quantitative syringe 22 to discharge from the needle 20 at least part of the sucked blood sample into a predetermined chamber 31 in the blood analysis portion 30 (S6, S7; a discharging step).

For example, if the sample container 10 is of a closed type, the control portion 40 drives the quantitative syringe 22 to push out from the needle 20 the blood sample into the cleaning container 32 (see Fig. 2) by an amount equal to or smaller than the predetermined amount Vm (S6). That is, part of the blood sample is discarded. Then, the control portion 40 discharges from the needle 20 the blood sample into the chamber 31 by the defined amount Vs (S7). By contrast, if the sample container 10 is of an open type, the control portion 40 drives the quantitative syringe 22 to discharge from the needle 20 the blood sample into the chamber 31 by the defined amount Vs (equal to the suction amount V) (S7). The amount of blood sample to be discharged from the needle 20 can be easily controlled by controlling the duration for which the control portion 40 drives the quantitative syringe 22.

Lastly, the blood analysis portion 30 analyzes the blood sample discharged into the chamber 31 (S8; an analyzing step).

As described above, based on container information, the control portion 40 judges whether the suction amount V of the blood sample with the needle 20 needs to be adjusted from the prescribed defined amount Vs and, based on the judgment result, sets the suction amount (S2, S3, S4-1, S4-2). In this way, regardless of the type of sample container 10 (a closed type or an open type), it is possible to discharge the defined amount Vs of blood sample from the needle 20 into the chamber 31 in the blood analysis portion 30 and to analyze the blood sample accurately. The effect of this will be described in more detail below.

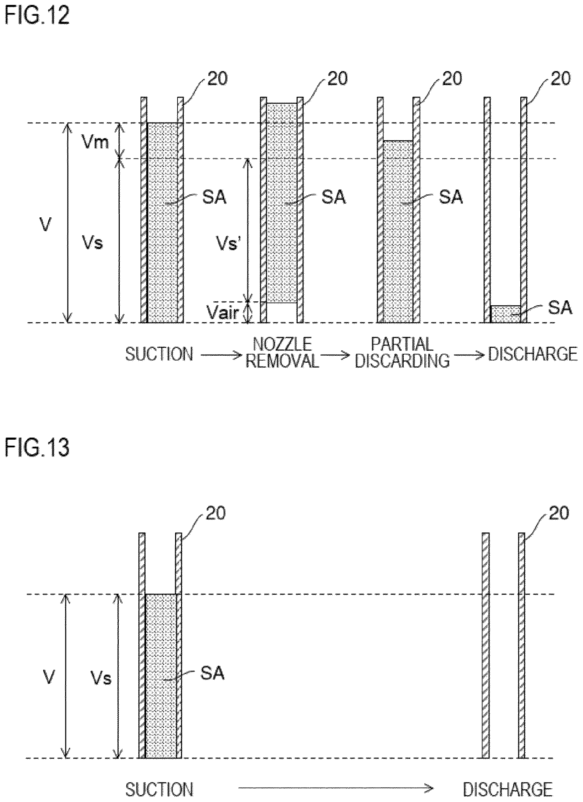

Fig. 12 schematically shows the variation of the blood sample SA in the needle 20 collected from the sample container 10P of a closed type. Fig. 13 schematically shows the variation of the blood sample SA in the needle 20 collected from the sample container 10Q of an open type. In Figs. 12 and 13, the top side is the base end side of the needle, and the bottom side is the tip end side of the needle.

As shown in Fig. 12, the blood sample SA, of which the suction amount V (= Vs + Vm) has been sucked from the sample container 10P into the needle 20, is, after the needle 20 is pulled out of the sample container 10P, pushed toward the base end of the needle due to a pressure variation between inside and outside the sample container 10P. Thus, air enters the tip end of the needle (the amount of air here is represented by Vair). In this state, when the quantitative syringe 22 is driven for a period corresponding to the discharging period for the defined amount Vs to discharge the blood sample SA from the needle 20 into the chamber 31, the amount of blood sample SA discharged from the needle 20 is reduced by the amount of air Vair that has entered the tip of the needle, down to an amount Vs' that is smaller than the defined amount Vs (Vs = Vs' + Vair). Thus, due to a supply of the blood sample SA insufficient for blood analysis, the blood analysis portion 30 cannot perform accurate blood analysis.

However, in this embodiment, in S6, a predetermined amount Vm or less of blood sample SA is pushed out to be discarded from the needle 20; thus, the air that has entered the tip of the needle is pushed out so that the blood sample SA is present in the tip end of the needle. Thus, in this state, by driving the quantitative syringe 22 for a period corresponding to the discharging period for the defined amount Vs, the defined amount Vs of blood sample SA can be discharged from the needle 20 into the chamber 31. As a result, the blood analysis portion 30 can perform accurate blood analysis using the defined amount Vs of blood sample SA . After the defined amount Vs of blood sample SA is discharged, the blood sample SA remaining in the needle 20 is, through the cleaning of the needle 20, disposed of into the waste liquid container 50 (see Fig. 2).

On the other hand, as shown in Fig. 13, when the blood sample SA is sucked into the needle 20 by the suction amount V (= Vs) from the sample container 10Q of an open type, even when the needle 20 is removed from the sample container 10Q, no pressure variation as in the case of the sample container of a closed type occurs; thus, no displacement of the blood sample SA contained in the needle 20 occurs. Thus, in this state, by driving the quantitative syringe 22 for a period corresponding to the discharging period for the defined amount Vs, the defined amount Vs of blood sample SA can be discharged from the needle 20 into the chamber 31. As a result, the blood analysis portion 30 can perform accurate blood analysis using the defined amount Vs of blood sample SA .

If the sample container 10 is of a closed type (first type), the control portion 40 judges that the suction amount V needs to be adjusted from the defined amount Vs and sets the suction amount V to an amount larger than the defined amount Vs by the predetermined amount Vm (S3, S4-1). If the sample container 10 is of a closed type, as described above, due to a pressure difference that arises when, after the suction of the blood sample, the needle 20 is pulled out of the sample container 10P, the defined amount of blood sample may not be discharged into the chamber 31 in the blood analysis portion 30. Thus, the control described above by the control portion 40 is effective particularly when the sample container 10P of a closed type is used.

The control portion 40 pulls the needle 20 out of the sample container 10, then pushes out the blood sample inside the needle 20 from the tip end of the needle by the predetermined amount Vm or less, then discharges the blood sample from the needle 20 into the chamber 31 by the defined amount Vs (S5-1, S6). In this case, with the blood sample present in the tip of the needle, the defined amount Vs of blood sample can be discharged from the needle 20 into the chamber 31.

When the sample container 10 is of an open type (second type), the control portion 40 judges that there is no need to adjust the suction amount V from the defined amount Vs, and sets the suction amount V to the same amount as the defined amount Vs (S3, S4-2). When the sample container 10 is of an open type, as described above, no problem as in the case of the sample container 10P of a closed type occurs. Thus, when using the sample container 10Q of an open type, setting the suction amount V to the defined amount Vs and discharging into the chamber 31 in the blood analysis portion 30 the defined amount Vs of blood sample sucked from the needle 20 permits the blood analysis portion 30 to perform accurate blood sample analysis.

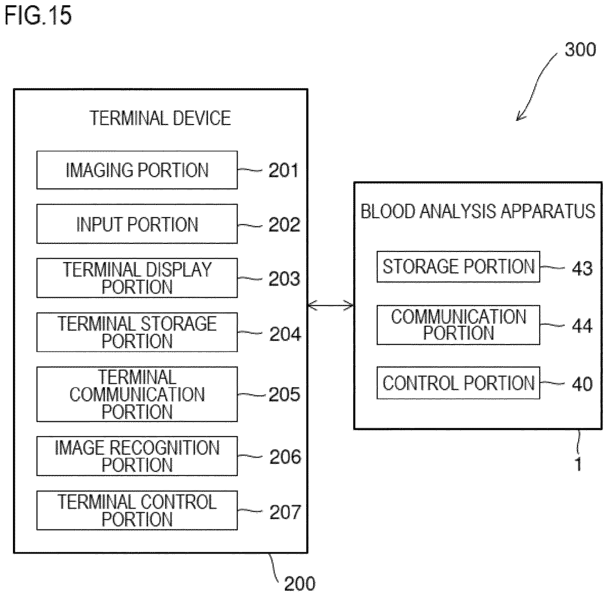

While, in the procedure described above, the control portion 40 judges whether the suction amount of the blood sample with the needle 20 needs to be adjusted from the defined amount based on the container information acquired in S2 (S3) and sets the suction amount based on the judgment result (S4-1, S4-2), this is not meant to be any limitation. For example, the storage portion 43 (see Fig. 15) may store a table predefining the relationship between types of sample container 10 (open type, closed type) and suction amounts, and the control portion 40 may, based on container information acquired in S2, read from the storage portion 43 the suction amount corresponding to the type of the sample container 10 and set it (S4-1, S4-2).

### [ 6. Another Configuration of a Blood Analysis Apparatus ]

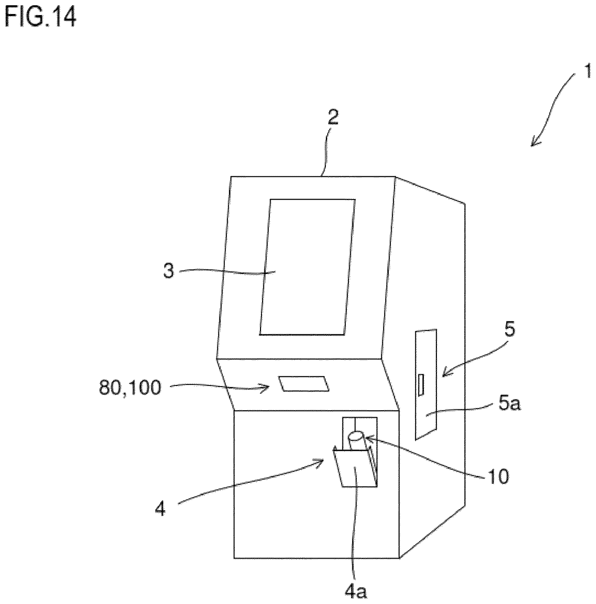

Fig. 14 is a perspective view showing another configuration of the blood analysis apparatus 1 according to the embodiment. In the front face of the apparatus body 2, between the display portion 3 and the sample container loading portion 4, an information reading portion 80 may be provided. The information reading portion 80 is configured with, for example, a bar code reader, and optically reads identification information printed on the label L (see Figs. 3 and 4) attached to the side surface of the sample container 10. As described above, the identification information includes information on the type of sample container 10 (closed type, open type). The information reading portion 80 may be configured to read a two-dimensional code.

In the information acquisition step in S2 described above, the information reading portion 80 may read identification information unique to the sample container 10 to acquire container information on the type of sample container 10. Also by reading the identification information with the information reading portion 80, it is possible to acquire container information reliably. Thus, in this case, it can be said that the information reading portion 80 functions as the container information acquisition portion 100.

If the blood analysis apparatus 1 incorporates the information reading portion 80, compared with a case where an externally connected bar code reader is used, the information reading portion 80 can be made to read information even from a small space or with the sample container 10 held in one hand.

### [ 7. Blood Analysis System ]

Fig. 15 is a block diagram showing an outline of the configuration of a blood analysis system 300 according to the embodiment. The blood analysis system 300 is configured to include a terminal device 200 and the blood analysis apparatus 1 described above, with these connected such that they can communicate with each other via a communication network. The communication network may be wired or wireless.

The blood analysis apparatus 1 includes, in addition to the components in the embodiment described above, a storage portion 43 and a communication portion 44. The storage portion 43 is a memory that stores an operation program for the control portion 40 as well as various kinds of information, and is configured to include a ROM (read-only memory), a RAM (random-access memory), a non-volatile memory, and the like. The communication portion 44 is an interface for communication with the outside (for example, the terminal device 200) and is configured to include an antenna, a transmission/reception circuit, a modulation circuit, a demodulation circuit, and the like.

The terminal device 200 is a kind of computer such as a smartphone or a tablet computer. The terminal device 200 is configured to include an imaging portion 201, an input portion 202, a terminal display portion 203, a terminal storage portion 204, a communication portion 205, an image recognition portion 206, and a terminal control portion 207.

The imaging portion 201 is configured with, for example, a camera and acquires images by shooting. The imaging portion 201 can capture identification information such as a bar code to read the identification information. In this case, the imaging portion 201 functions as a terminal-side reading portion that reads identification information. The input portion 202 is configured with switches, buttons, and the like and accepts input of various kinds of information from the owner of the terminal device 200. The terminal display portion 203 is configured with, for example, a liquid crystal display device and displays various kinds of information. The terminal display portion 203 may be configured with a display device with a touch panel. In this case, the terminal display portion 203 can serve also as the input portion 202. The terminal storage portion 204 is a memory that stores an operation program for the terminal control portion 207 as well as various kinds of information, and is configured to include a ROM, a RAM, a non-volatile memory, and the like. The terminal communication portion 205 is an interface for communication with the outside (for example, the blood analysis apparatus 1) and is configured to include an antenna, a transmission/reception circuit, a modulation circuit, a demodulation circuit, and the like.

The image recognition portion 206 recognizes, from the image captured in the imaging portion 201, the shape of an object captured (for example, the adapters 61 and 62). Here, the shape may be recognized, for example, through pattern matching. The terminal control portion 207 is a CPU that, according to the operation program stored in the terminal storage portion 204, controls the operation of different parts in the terminal device 200.

Next, operation in the blood analysis system 300 will be described. First, in the terminal device 200, a predetermined program (application software) is downloaded and stored in the terminal storage portion 204. Next, the terminal control portion 207, based on container information on the type of sample container 10 to be used, sets the amount of blood sample to be sucked with the needle 20 (a suction amount setting step). Here, the container information mentioned above may be acquired by the image recognition portion 206 recognizing the shape of an adapter from the image obtained by the imaging portion 201 imaging at least one adapter (for example, the adapter 61 or 62) determined according to the type of sample container 10. The container information mentioned above may be acquired by the imaging portion 201, acting as a terminal-side reading portion, capturing and reading the identification information (e.g., bar code) attached to the sample container 10.

For example, if the image recognition portion 206 recognizes the shape of the adapter 61, the terminal control portion 207 judges that the sample container 10 in use is a sample container 10P of a closed type. The terminal control portion 207 then sets the suction amount V of the blood sample with the needle 20 to an amount larger than the defined amount Vs by a predetermined amount Vm. By contrast, for example, if the image recognition portion 206 recognizes the shape of the adapter 62, the terminal control portion 207 judges that the sample container 10 in use is a sample container 10Q of an open type. The terminal control portion 207 then sets the suction amount V of the blood sample to be sucked with the needle 20 to the same amount as the defined amount Vs.

Next, the terminal device 200 outputs information on the suction amount V set by the terminal control portion 207 to the blood analysis apparatus via the terminal communication portion 205 (an output step). In this way, the blood analysis apparatus 1 can suck the set suction amount V of the blood sample from the needle 20 and discharge at least part of the sucked blood sample into a predetermined chamber 31 in the blood analysis portion 30 to perform blood analysis.

Also with the configuration where, as described above, the suction amount V with the needle 20 is set by the terminal device 200 and the information on the set suction amount V is output from the terminal device 200 to the blood analysis apparatus 1, the blood analysis apparatus 1 can suck the set suction amount V of the blood sample according to the type of sample container 10 in use; thus, as in the embodiment, it is possible to accurately analyze the blood sample regardless of the type of sample container 10 in use.

### [ 8. Program ]

The control portion 40 in the blood analysis apparatus 1 can be configured with a computer with an operation program installed on it. The computer can read and execute the program to make different parts of the blood analysis apparatus 1 operate to perform the procedure (steps) described above. Such a program is obtained, for example, by being downloaded from outside via a network and is stored in the storage portion 43. The program may be read from a computer-readable recording medium such as a CD-ROM (compact read-only memory) by a reading portion (not shown) and the read program may be stored in the storage portion 43. That is, the program of this embodiment is a program for making a computer execute the blood analysis method described above.

Also the terminal control portion 207 in the terminal device 200 can be configured with a computer with an operation program installed on it. The computer can read and execute the program described above to make different parts in the terminal device 200 to operate to perform the procedure (step) described above. Such a program is obtained, for example, by being downloaded from outside via a network and is stored in the terminal storage portion 204.

The description of embodiments of the present invention given above is in no way meant to limit the invention, and various modifications are possible without departing from the spirit of the present invention.

### Industrial Applicability

The present invention finds applications in, for example, apparatuses for analyzing blood using a sample container of a closed type or of an open type.

### Reference Signs List

- 1: blood analysis apparatus
- 10, 10P, 10Q: sample container
- 10b: lid
- 20: needle
- 30: blood analysis portion
- 31: chamber
- 40: control portion
- 61: adapter
- 62: adapter
- 70: adapter sensing portion
- 80: information reading portion
- 100: information acquisition portion
- 201: imaging portion (terminal-side reading portion)

## Claims

1. A blood analysis apparatus comprising:
a container information acquisition portion that acquires container information on a type of a sample container to be used;
a needle that sucks and discharges a blood sample contained in the sample container;
a blood analysis portion having a chamber that receives the blood sample sucked from the sample container and discharged thereinto with the needle; and
a control portion that controls suction and discharge of the blood sample with the needle based on the container information,
wherein
the control portion sets an amount of the blood sample to be sucked with the needle based on the container information.

2. The blood analysis apparatus according to claim 1,
wherein
the control portion judges whether the suction amount needs to be adjusted from a prescribed defined amount based on the container information and sets the suction amount based on a judgment result.

3. The blood analysis apparatus according to claim 2,
wherein
when the sample container is of a first type, the control portion judges that the suction amount needs to be adjusted from the defined amount and sets the suction amount to an amount larger than the defined amount by a predetermined amount, and
the first type is a closed type in which the needle pierces a lid of the sample container to suck the blood sample contained in the sample container.

4. The blood analysis apparatus according to claim 3,
wherein
the control portion pulls the needle out of the sample container, then pushes out the blood sample inside the needle from a tip end of the needle by the predetermined amount or less, and then discharges the blood sample from the needle into the chamber by the defined amount.

5. The blood analysis apparatus according to claim 3 or 4,
wherein
when the sample container is of a second type, the control portion judges that there is no need to adjust the suction amount from the defined amount and sets the suction amount to a same amount as the defined amount, and
the second type is an open type in which, with a lid of the sample container removed, the needle is inserted into the sample container to suck the blood sample directly.

6. The blood analysis apparatus according to any one of claims 1 to 5,
wherein
the container information acquisition portion includes an adapter sensing portion that, when the sample container is attached to at least one adapter determined according to the type of the sample container and is loaded in an apparatus body, acquires the container information by sensing the adapter.

7. The blood analysis apparatus according to any one of claims 1 to 6,
wherein
the container information acquisition portion includes an information reading portion that acquires the container information by reading identification information that is attached to the sample container and that is unique to the sample container.

8. A blood analysis method comprising:
a suction amount setting step of setting an amount of a blood sample to be sucked with a needle based on container information on a type of a sample container to be used;
a suction step of sucking with the needle the set suction amount of the blood sample from the sample container;
a discharging step of discharging at least part of the sucked blood sample from the needle into a chamber in a blood analysis portion; and
an analyzing step of analyzing the blood sample discharged in the chamber.

9. The blood analysis method according to claim 8,
wherein
in the suction amount setting step,
it is judged whether the suction amount needs to be adjusted from a prescribed defined amount based on the container information, and
the suction amount is set based on a judgement result.

10. The blood analysis method according to claim 9,
wherein
in the suction amount setting step, when the sample container is of a first type, it is judged that the suction amount needs to be adjusted from the defined amount and the suction amount is set to an amount larger than the defined amount by a predetermined amount, and
the first type is a closed type in which the needle pierces a lid of the sample container to suck the blood sample contained in the sample container.

11. The blood analysis method according to claim 10,
wherein
in the suction step, the needle is taken out of the sample container after the blood sample is sucked, and
in the discharging step, the blood sample is pushed out from the needle by the predetermined amount or less, then the blood sample is discharged from the needle into the chamber by the defined amount.

12. The blood analysis method according to claim 10 or 11,
wherein
in the suction amount setting step, when the sample container is of a second type, it is judged that there is no need to adjust the suction amount from the defined amount and the suction amount is set to a same amount as the defined amount, and
the second type is an open type in which, with a lid of the sample container removed, the needle is inserted into the sample container to suck the blood sample directly.

13. The blood analysis method according to any one of claims 8 to 12,
wherein
the container information is acquired, when the sample container is attached to at least one adapter determined according to the type of the sample container and is loaded in an apparatus body, by a sensing portion sensing the adapter.

14. The blood analysis method according to any one of claims 8 to 12,
wherein
the container information is acquired by an information reading portion reading identification information that is attached to the sample container and that is unique to the sample container.

15. A program for making a computer execute the blood analysis method according to any one of claims 8 to 14.

16. A program for making a computer execute:
a suction amount setting step of setting an amount of a blood sample to be sucked with a needle based on container information on a type of a sample container to be used, and
an output step of outputting information on the set suction amount to a blood analysis apparatus.

17. The program according to claim 16,
wherein
the container information is acquired by an image recognition portion recognizing, from an image obtained by an imaging portion imaging at least one adapter determined according to the type of the sample container, a shape of the adapter.

18. The program according to claim 16,
wherein
the container information is acquired by a terminal-side reading portion reading identification information that is attached to the sample container and that is unique to the sample container.
